# EUROPÄISCHE PATENTANMELDUNG

(11) **EP 3 882 324 A1**
(43) Veröffentlichungstag der Anmeldung: **22.09.2021**
(21) Anmeldenummer: 20163389.8
(22) Anmeldetag: 16.03.2020
(51) Int. Cl.: C09J 7/30, A61F 13/02, A61F 13/08, C09J 123/20

(54) **BANDAGE, HOTMELT-KLEBSTOFF ZUR HERSTELLUNG EINER BANDAGE, VERWENDUNG UND VERFAHREN ZUR HERSTELLUNG EINES HOTMELT-KLEBSTOFFS**

(71) Anmelder: artimelt AG, 6210 Sursee (CH)
(72) Erfinder: Nyffeler, Judith, 6017 Ruswil (CH); Senn, Nicole, 5726 Unterkulm (CH)
(74) Vertreter: Hepp Wenger Ryffel AG

(57) **Zusammenfassung**

Die Erfindung betrifft das technische Gebiet der kohäsiven Bandagen. Die Erfindung umfasst eine Bandage umfassend ein Flachbahnmaterial als Substrat wobei beide Hauptseiten des Substrats zumindest teilweise mit einem Hotmelt-Klebstoff beschichtet sind; weiter auf eine Hotmelt Klebstoff zur Herstellung einer Bandage; weiter auf eine Verwendung eines Hotmelt-Klebstoffs zur Herstellung von Bandagen und auf ein Verfahren zur Herstellung einer Bandage.

Der Hotmelt-Klebstoff basiert auf einem ersten Polyolefin, aufweisend
- einen Anteil von 1-Buten abgeleiteten Monomer-Einheiten;
- einem Anteil von Ethylen abgeleiteten Monomer-Einheiten;
und wobei der Hotmelt-Klebstoff weiter umfasst:
- wenigstens einen Klebrigmacher aus Kohlenwasserstoffharz und/oder aus hydriertem Kohlenwasserstoffharz.

Ferner kann der Hotmelt-Klebstoff ein zweites Polyolefin aufweisen, bevorzugt ein amorphes Polypropylen Homo- oder Copolymer.

## Beschreibung

Die Erfindung betrifft das technische Gebiet der kohäsiven Bandagen. Kohäsive Bandagen sind auf sich selbst haftende Fixierbinden für die Fixierung von Wundverbänden. Sie finden Anwendung als therapeutische und prophylaktische Kompressions-, Stütz- oder Schutzverbände beim Sport, in der Orthopädie, Chirurgie oder im Alltag.

Kohäsive Bandagen müssen verschiedene Anforderungen erfüllen. Sie müssen gut auf sich selbst haften, rutschfest sein, v.a. für den Einsatz an vielbewegten Körperteilen, sowie hautfreundlich und luftdurchlässig sein.

Traditionell wurde zur Herstellung von kohäsiven Binden ein flexibles Textil mit Naturkautschuk aus Latex verwendet. Naturkautschuk hat aber allergene Eigenschaften, die einen beträchtlichen Teil der Bevölkerung betreffen. Daher wurde Naturkautschuk zusehends mit synthetischem Kautschuk ersetzt.

Die WO 2017/109210 offenbart eine Bandage aus elastischem Flachbahnmaterial als Substrat, welches auf beiden Seiten mit Synthesekautschuk (Polyisopren, Polybutadien, Polychloropren) beschichtet oder getränkt wird. Im genannten Beispiel besteht der kohäsive Klebstoff aus 1.4-Polyisopren (NATSYN 2200), welcher mit einem Alterungsschutzmittel versehen und in Lösung aufgetragen wird. Die Haftung des verwendeten Natsyn bleibt jedoch deutlich hinter der Haftung von Naturkautschuk zurück, was sich negativ auf den Tragekomfort aber auch auf die Leistungsfähigkeit von Verbänden, insbesondere Kompressionsverbänden auswirkt. Es besteht daher ein Bedarf nach kohäsiven Bandagen, welche wenig allergen sind, sich von der Haftleistung her jedoch ähnlich wie Naturkautschuk verhalten.

Es ist die Aufgabe der vorliegenden Erfindung, die vorbeschriebenen Nachteile des Stands der Technik zu überwinden. Insbesondere soll eine kohäsive Bandage vorgeschlagen werden, welche zuverlässige Haftung auf sich selbst bietet, vergleichbar mit derjenigen von Naturkautschuk, ohne jedoch allergische Reaktionen auszulösen.

Diese Aufgabe wird gelöst durch eine Bandage, ein Hotmelt-Klebstoff zur Herstellung einer Bandage sowie eine Verwendung und ein Verfahren zur Herstellung eines Hotmelt-Klebstoffs gemäss den unabhängigen Patentansprüchen.

Die erfindungsgemässe Bandage umfasst ein Flachbahnmaterial als Substrat, wobei beide Hauptseiten des Substrats zumindest teilweise mit einem Hotmelt-Klebstoff beschichtet sind, insbesondere mit einem Hotmelt-Klebstoff aufweisend ein erstes Polyolefin aus:
- einem Anteil von 1-Buten abgeleiteten Monomer-Einheiten;
- einem Anteil von Ethylen abgeleiteten Monomer-Einheiten;
und wobei der Hotmelt-Klebstoff weiter umfasst:
- wenigstens einen Klebrigmacher aus Kohlenwasserstoffharz und/oder aus hydriertem Kohlenwasserstoffharz.

Unter einem Flachbahnmaterial wird hiernach verstanden ein flexibles, bandförmiges Substrat. Die Hauptseiten des Flachbahnmaterials sind die zwei einander gegenüberliegenden Seiten, welche die grösste Flächenausdehnung aufweisen. Wenn die Bandage bei bestimmungsgemässer Verwendung am menschlichen Körper angebracht ist, zeigt die innere Hauptseite gegen den menschlichen Körper, während die äussere Hauptseite vom menschlichen Körper wegweist.

Die innere Hauptseite kann zumindest Abschnittsweise direkt auf der menschlichen Haut aufliegen, sie kann zumindest Abschnittsweise auf einem Wundverband aufliegen und/oder zumindest Abschnittsweise auf der äusseren Hauptseite derselben Bandage oder der äusseren Hauptseite einer zweiten Bandage aufliegen. Typischerweise wird die Bandage mehrfach um ein Körperteil, z.B. eine Extremität, gewickelt, wobei die Auflageabschnitte der inneren Bandagenhauptseiten auf den äusseren Bandagenhauptseiten (Siegelflächen) die Haftung der Verbandsanordnung gewährleisten oder unterstützten.

Der Erfindung liegt die Idee zugrunde, dass es wünschenswert ist, wenn die Hauptseiten der Bandagen eine hohe Haftung aufeinander aufweisen und gleichzeitig die Hauptseiten der Bandagen auf anderen Oberflächen als den Hauptseiten der Bandagen (z.B. auf der Haut oder der Kleidung eines Patienten) eine besonders niedrige Haftung aufweisen. Als Haftung wird insbesondere das Abschneiden eines Materials im T-Peel Test verstanden.

Üblicherweise wird die Haftung als Zusammenspiel aus Adhäsion, Kohäsion und Tack dargestellt. Dabei bezieht sich Tack auf die Geschwindigkeit, mit der eine adhäsive Bindung hergestellt wird, und Adhäsion auf die Verbundstärke, welche zwischen dem Klebstoff und der zu verklebenden Oberfläche erzielt wird (physikalischen, makroskopisch beobachtbaren Anziehung). Klebstoffe sind häufig so beschaffen, dass sie einen hohen Tack und eine hohe Adhäsion aufweisen, die sich aber gegen eine Vielzahl von Partneroberflächen richten kann. Dies ist im Fall einer kohäsiven Bandage nicht wünschenswert. Die kohäsive Bandage soll insbesondere nicht an der Haut haften, um ein schmerzfreies Entfernen des Verbands zu gewährleisten. Auch soll die kohäsive Bandage nicht an anderen Oberflächen haften, beispielsweise an Kleidungsstücken, die über dem Verband getragen werden. Eine solche Haftung würde mit der Haftung der Bandage auf sich selber in Wettbewerb treten und Kräfte ausüben, die die Bandage zum Verrutschen oder Ablösen bringen.

Die erfindungsgemässe Bandage trägt dieser Problematik Rechnung. Das eingesetzte erste Polyolefin gewährleistet eine hohe Haftstärke wenn Hauptseite gegen Hauptseite gerichtet wird, geht aber keine störenden Klebeverbindungen mit anderen Oberflächen ein. Die detaillierten Werte werden nachfolgend angegeben. Aufgrund des oder der eingesetzten Klebrigmacher(s) aus Kohlenwasserstoffharz und/oder aus hydriertem Kohlenwasserstoffharz stellt sich die gewünschte Haftverbindung zwischen zwei Hauptseiten des Substrats auch rasch ein, ohne dass dafür übermässige Druckeinwirkung nötig wäre. Auf die Verwendung von Klebestreifen oder Klemmen kann dank der Eigenschaften der erfindungsgemässen Bandage verzichtet werden.

Ein Aspekt der Erfindung bezieht sich auf eine Bandage wie vorstehend beschrieben, wobei der Hotmelt-Klebstoff wenigstens ein zweites Polyolefin aufweist, wobei das zweite Polyolefin bevorzugt amorph ist, besonders bevorzugt Propylen-basiert ist, besonders bevorzugt eine Mischung aus Propylen-Homopolymer und Propylen-basiertem Copolymer ist. Beispielsweise kann das zweite Polyolefin ein Blend sein aus einem Propylen-Homopolymer und einem Copolymer aufweisend aus Propylen und aus Ethylen abgeleitete Monomer-Einheiten. Ein solches zweites Polyolefin hat bevorzugt ein niedriges Molekulargewicht. Eine typische Schmelzviskosität beträgt 1500 bis 2500 mPa·s, bevorzugt von 1800 bis 2200 mPa·s (bei 190°C). Ein solches zweites Polyolefin ist weich und klebrig und verbessert so zusätzlich die Flexibilität, aber auch die Haftungseigenschaften der Bandage.

Es ist bevorzugt, dass die kombinierten Anteile des ersten und zweiten Polyolefins am Hotmelt-Klebstoff insgesamt 17 bis 43 Gew.-%, bevorzugt 20 bis 42 Gew.-%, besonders bevorzugt 25 bis 40 Gew.-% betragen. Durch einen solchen Polyolefin-Anteil wird die zuvor beschriebene Haftungsstärke der kohäsiven Binde auf sich selbst optimiert. Es ist bevorzugt, dass dabei der Anteil des ersten Polyolefins aus einem Anteil von 1-Buten abgeleiteten Monomer-Einheiten und einem Anteil von Ethylen abgeleiteten Monomer-Einheiten 8 bis 25 Gew.-%, bevorzugt 10 bis 20 Gew.-%, am Totalgewicht des Hotmelt-Klebstoffs beträgt.

Ein Aspekt der Erfindung bezieht sich auf eine Bandage wie vorstehend beschrieben, wobei im Hotmelt-Klebstoff das anteilige Verhältnis (in Gew.-%) von Klebrigmacher zum Gesamtanteil von Polyolefin im Bereich von 0.6 bis 3, bevorzugt im Bereich von 0.8 bis 1.8, noch mehr bevorzugt im Bereich von 1 bis 1.4 liegt.

Es ist bevorzugt, wenn der Hotmelt-Klebstoff einen Schmiermittelzusatz umfasst, bevorzugt ein Wachs, besonders bevorzugt ein Polypropylenwachs. Der relative Anteil des Schmiermittelzusatzes an der Gesamtmasse des Hotmelt-Klebstoffs beträgt bevorzugt wenigstens 10 Gew.-%, besonders bevorzugt 15-40 Gew.-% und noch mehr bevorzugt 20-35 Gew.-%.

Da der Hotmelt-Klebstoff zur Herstellung der Bandage auf ein flexibles, insbesondere dehnbares Substrat aufgetragen wird, muss Viskosität sowohl im Auftragungsschritt als auch im fertigen Produkt gewährleistet sein. Dies wird durch die Verwendung eines relativ hohen Schmiermittelanteils gewährleistet.

Es ist bevorzugt, dass der Anteil von 1-Buten abgeleiteten Monomer-Einheiten im ersten Polyolefin 80 bis 92 Gew.-% beträgt, bevorzugt 84 bis 90 Gew.-% beträgt. Besonders bevorzugt handelt es sich um ein Buten-1/Ethylen-Copolymer, sodass der Ethylen-Monomer Anteil den 1-Buten-Monomer Anteil auf 100% ergänzt. Es ist bevorzugt, dass das erste Polyolefin eine Melt Flow Rate (MFR) von 0.5 bis 7 g / 10 min, bevorzugt 1.0 bis 3 g/10min, besonders bevorzugt ca. 1.3g / 10 min hat, messbar nach DIN EN ISO 1133-1:2012-03 (190°C; 2.16kg).

Eine Oberfläche, die mit einem solchen ersten Polyolefin beschichtet ist, hat eine besonders hohe Haftung zu einer Partneroberfläche, die mit demselben ersten Polyolefin beschichtet ist (Siegelflächen). Die beiden Partneroberflächen sind leicht (von Hand) voneinander abziehbar, können aber auch leicht wieder zusammengefügt werden. Sie haben eine nahezu konstante Peel-Festigkeit, auch beim wiederholten Gebrauch. Der Begriff "peelbar" bezieht sich auf eine Trennung an der Grenzfläche der Siegelfläche. Es ist ein besonderer Vorteil eines solchen ersten Polyolefins dass es die vorteilhaften Eigenschaften, also Peelbarkeit und Haftung von Partnerflächen aufeinander, bei Raumtemperatur (22°C ±5°C) aufweist.

Es ist bevorzugt, dass der Hotmelt-Klebstoff eine Schmelzviskosität von 1'000 bis 50'000 mPa·s hat, bevorzugt 10'000 bis 45'000 mPa · s, besonders bevorzugt 15'000 bis 30'000 mPa ·s, messbar nach EN ISO 3219 (Cone: 35mm/2°; Drehzahl = 31.6 sec⁻¹; 160°C). Ein Hotmelt-Klebstoff aufweisend diese Schmelzviskosität lässt sich besonders gut compoundieren und kann problemlos auf das Substrat aufgetragen werden. Die Viskosität ist wichtig für den gleichmässigen, einfachen und dennoch gut haftenden Auftrag. Wenn der Hotmelt-Klebstoff eine allzu hohe oder allzu niedrige Viskosität aufweisen würde, müsste die Viskosität noch mittels Lösungsmittelzusätzen eingestellt werden um den konventionellen Auftrag (Sprühauftrag, Aufdruck, Tauchen, Messer- oder Walzenrakeln oder Giessverfahren) zu ermöglichen. Insbesondere wenn organische Lösungsmittel verwendet werden, belastet eine solche Formulierung die Umwelt und erfordert Massnahmen zum Sicherstellen der Arbeitssicherheit. Ausserdem besteht dann die Gefahr, dass die Verankerung des Materials im Substrat, insbesondere einem textilen Substrat, unzureichend ist. Bei allzu hoher Viskosität wird ein aufwändigeres Verfahren durch Düsenextrusion notwendig. Ein Hotmelt-Klebstoff, der aber eine Schmelzviskosität wie oben beschrieben hat, umgeht diese Probleme und lässt sich trotz Verzichts auf Lösungsmittel gut durch Breitschlitzdüsen direkt auf das Substrat auftragen wo er sehr gut verankert.

Es ist bevorzugt, dass das erste Polyolefin eine Glasübergangstemperatur von -27°C hat, messbar nach DMTA Analyse. Ein Polyolefin aufweisend eine solche Glasübergangstemperatur weist bei Raumtemperatur hinreichend kristalline Anteile auf, sodass die erforderliche Klebrigkeit im fertigen Produkt erreicht wird.

In der DMTA (dynamisch-mechanische Thermo-Analyse) werden Gussproben von 76mm x 13mm x 1 mm in der DMTA-Maschine fixiert zum Ausüben einer Zugbeanspruchung. Die Frequenz von Zug und Entspannung der Probe wird auf 1 Hz eingestellt. Die DMTA überträgt die elastische Reaktion der Probe von -100°C auf 130°C. Auf diese Weise ist es möglich, die elastische Reaktion gegenüber der Temperatur aufzutragen. Der Elastizitätsmodul für ein viskoelastisches Material ist definiert als E = E'+iE". Die DMTA kann die beiden Komponenten E' und E" aufgrund ihrer Resonanz aufspalten und E' gegen die Temperatur aufzeichnen. Ebenso können E`+E"=tan(δ) gegen die Temperatur aufgezeichnet werden. Die Glasübergangstemperatur Tg wird als die Temperatur am Maximum der Kurve E'+E"=tan(δ)gegen die Temperatur abgelesen.

Ein Aspekt der Erfindung bezieht sich auf eine Bandage wie vorstehend beschrieben, wobei der Hotmelt-Klebstoff eine Filmfestigkeit von 0.5 bis 1.5 N/mm² aufweist, bevorzugt von 0.6 bis 1.1 N/mm², messbar gemäss DIN 53504. Ein weiterer Aspekt der Erfindung bezieht sich auf eine Bandage wie vorstehend beschrieben, wobei der Hotmelt-Klebstoff eine Bruchdehnung von 700 bis 1200%, bevorzugt eine Bruchdehnung von 800 bis 1000% aufweist, messbar gemäss DIN 53504. Eine Bandage, welche eine hohe Filmfestigkeit und Bruchdehnung hat, eignet sich besonders für die Herstellung kohäsiver Binden, da so die im Alltag auf die Bandage wirkenden Kräfte die Integrität des Verbands nicht beeinträchtigen. Eine solche Bandage hat eine hohe Dehnbarkeit. Der Hotmelt-Klebstoff, der z.B. auf ein elastisches textiles Flachbahnmaterial aufgetragen ist, kann sich ohne Rissbildung zusammen mit dem Substrat ausdehnen und wieder zusammenziehen, was für kohäsive Binden besonders vorteilhaft ist.

Wie bereits eingangs beschrieben, sollte die Bandage hautfreundlich und luftdurchlässig sein und gegebenenfalls eine Kompressionswirkung auf das zu behandelnde Körperteil ausüben können. Das Substrat kann daher z.B. ein Gewebe, Gewirke, Gestricke oder ein Vlies sein. Das Substrat der Bandage wie vorstehend beschrieben ist bevorzugt ein gewebtes oder nicht-gewebtes Textil, bevorzugt ein in Längs- und/oder Querrichtung elastisches gewebtes oder nicht-gewebtes Textil. Beispielsweise kann eine elastische Gewebekonstruktion aus der Kombination von dauerelastischen Elastanfäden mit starren Baumwollgarnen resultieren.

Ein weiterer Aspekt der Erfindung betrifft einen Hotmelt-Klebstoff zur Herstellung einer Bandage wie vorstehend beschrieben, wobei der Hotmelt-Klebstoff auf einem ersten Polyolefin basiert, aufweisend
- einen Anteil von 1-Buten abgeleiteten Monomer-Einheiten;
- einem Anteil von Ethylen abgeleiteten Monomer-Einheiten;
und wobei der Hotmelt-Klebstoff weiter umfasst:
- wenigstens einen Klebrigmacher aus Kohlenwasserstoffharz und/oder aus hydriertem Kohlenwasserstoffharz;
wobei die Schälfestigkeit eines Verbunds aus zwei mit dem Hotmelt-Klebstoff beschichteten Substraten bei Raumtemperatur wenigstens 1 bis 10 N/25mm, vorzugsweise 1.5 bis 6.5 N/25mm, besonders bevorzugt 4 bis 6 N/25mm beträgt, messbar nach ASTM D 1876-01.

Wo nicht explizit etwas anderes Beschrieben ist, sind die Vorgaben von Messmethode ASTM D 1876-01 anwendbar. Allfällige Abweichungen gehen aus den nachfolgenden Ausführungsbeispielen (Beispiel 3) hervor. Ein Hotmelt-Klebstoff, der die besagte Schälfestigkeit aufweist, eignet sich zur Herstellung einer Bandage, die hilfsmittelfrei, also ohne die Verwendung von z.B. Klebestreifen oder Klammern, als Schutz-, Wund- oder Kompressionsverband eingesetzt werden kann. Dabei wird der Halt des Schutz-, Wund- oder Kompressionsverbands durch die Haftung der Siegelflächen im hinreichenden Masse gewährleistet.

Es ist bevorzugt, wenn der Hotmelt-Klebstoff so beschaffen ist, dass die Schälfestigkeit eines Verbunds aus einem mit dem Hotmelt-Klebstoff beschichtetem Substrat auf Materialien, die nicht mit dem Hotmelt-Klebstoff beschichtet sind, bei Raumtemperatur minimal bis nicht-existent ist. Dies lässt sich mit qualitativen Tests beurteilen. Wie aus den nachfolgenden konkreten Ausführungsformen (Beispiel 4) hervorgeht, kann die Haftung von mit Klebstoff beschichteten Folien und Textilien auf der Haut von Testpersonen nach Ablauf einer gewissen Zeit subjektiv bewertet und das Resultat mit demjenigen für herkömmliche Klebstoffe verglichen werden.

Ein weiterer Aspekt der Erfindung bezieht sich auf die Verwendung eines Hotmelt-Klebstoffs wie vorstehend beschrieben zur Herstellung von Bandagen. Der Hotmelt-Klebstoff basiert auf einem ersten Polyolefin, insbesondere aufweisend
- einen Anteil von 1-Buten abgeleiteten Monomer-Einheiten;
- einen Anteil von Ethylen abgeleiteten Monomer-Einheiten;
wobei der Hotmelt-Klebstoff weiter umfasst:
- wenigstens einen Klebrigmacher aus Kohlenwasserstoffharz und/oder hydriertem Kohlenwasserstoffharz.
Es ist bevorzugt, dass der Hotmelt-Klebstoff sich zusammensetzt wie vorstehend beschrieben und der Hotmelt-Klebstoff respektive die die daraus hergestellten Filme und Bandagen die vorstehend beschriebenen Eigenschaften aufweisen.

Ein weiterer Aspekt der Erfindung bezieht sich auf ein Verfahren zur Herstellung einer Bandage, umfassend die folgenden Schritte:
- Bereitstellen eines Flachbahnmaterials als Substrat, insbesondere eines in Längs- und/oder Querrichtung elastischen gewebten oder nicht-gewebten Textils;
- Doppelseitiges Auftragen eines Hotmelt-Klebstoffs nach Anspruch 11 auf das Substrat.

Dabei kann das doppelseitige Auftragen des Hotmelt-Klebstoffs durch extrudieren, kaschieren, etc. erfolgen. Es ist bevorzugt, wenn der Hotmelt-Klebstoff lösungsmittelfrei bereitgestellt wird. Unter "lösungsmittelfrei" wird verstanden die Abwesenheit von wässrigen Phasen oder organischen Phasen, insbesondere von Alkoholen, Ethern, Aromaten, etc. Es ist bevorzugt, wenn der Hotmelt-Klebstoff mittels Extrusion durch Breitschlitzdüsen beidseitig auf das Flachbahnmaterial aufextrudiert wird.

Die Erfindung wird nachfolgend in Ausführungsbeispielen und anhand von Figuren näher erläutert, ohne dass die Erfindung auf diese konkreten Ausführungsbeispiele zu beschränken ist.

Es zeigt:

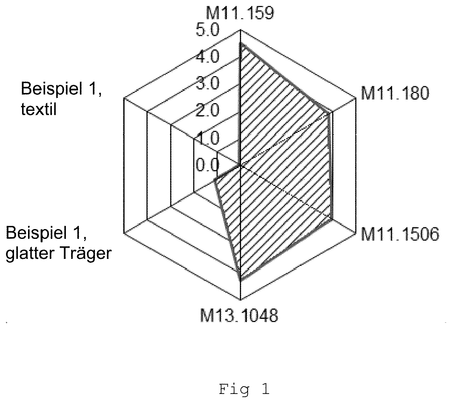

- Figur 1: Diagramm der Resultate aus der qualitativen Hauthaftungsprüfung gemäss Beispiel 4

### Beispiel 1:

Es wird ein Hotmelt-Klebstoff aus folgenden Bestandteilen gemischt:

| | Anteil (Gew.-%) |
|---|---|
| Compoundiertes Buten-1/Ethylen Co-Polymer (72 Gew.-% Buten-1; 13 Gew.-% Ethylen; 15 Gew.-% Klebrigmacher) | 15 |
| Amorphes Polyolefin (Blend aus Propylen-Homopolymer und Propylen-Ethylen-Copolymer, niedermolekular) | 20 |
| Kohlenwasserstoffharz (cycloaliphatisches Kohlenwasserstoffharz, amorph, niedermolekular, wasserhell) | 35 |
| Polypropylenwachs (niedermolekular, aus Metallocen-Katalyse) | 28 |
| Stabilisator | 2 |

### Beispiel 2:

Die Schmelzviskosität des Klebstoffs nach Beispiel 1 wurde nach EN ISO 3119 bestimmt und betrugt 22'000 mPa·s. Die Filmfestigkeit des Klebstoffs wurde nach DIN 53504 gemessen und betrug 0.9 N/mm. Die Bruchdehnung des Klebstoffs wurde ebenfalls nach DIN 35304 bestimmt und sie betrug 900%.

### Beispiel 3:

Es wurde eine Schälfestigkeitsprüfung gemäss ASTM D 1876-01 durchgeführt. Als Trägermaterial wurde eine unvorbehandelte 23my PET-Folie verwendet. Die Zusammensetzung aus Beispiel 1 wurde durch Breitschlitzdüsen direkt auf ein PET-Trägermaterial geschichtet. Die Dicke der Klebstoffschicht betrugt 12-15 g/cm² Die beschichteten Träger wurden 24 h bei 23°C und 50% rel. Luftfeuchtigkeit gelagert.

Die Proben wurden mit gegen einander weisenden Klebeseiten verklebt und leicht angedrückt. Aus dem Verbund wurde mittels Japanmesser ein Teststreifen von 100 x 25 mm geschnitten und mit 2 kg Gewicht zweimal in beide Richtungen angerollt. Danach wurde der Teststreifen nochmals 20 min gelagert. Die Dicke der resultierenden Haftschicht nach dem Verbindungsschritt betrug 24 bis 30 g/m². Pro Probenmaterial werden jeweils mindestens 4 Probenstreifen hergestellt. Die T-Peel Messung erfolgte auf einer Zugprüfmaschine von Instron, wobei die Greifarme auf eine Separationsgeschwindigkeit von 300 mm/min eingestellt wurden. Die Kraft wurde durch die Zugprüfmaschine über die gesamte Abzugsstrecke von 100 mm gemessen und es wurde der arithmetische Mittelwert über die Messstrecke (abzüglich die ersten und letzten 10% des Weges) errechnet.

Die maximalen, minimalen und durchschnittlichen Kräfte bei einem einzelnen Teststreifen betrugen 8N/25mm, 2N/25mm und 5N/25mm. Die über alle Proben gemittelte Durchschnittskraft pro Strecke belief sich auf 5N/25mm.

### Beispiel 4:

Eine qualitative Beurteilung der Haftung des Hotmelt-Klebstoffs und der Bandage beschichtet mit dem Hotmelt-Klebstoff auf menschlicher Haut wurde bei 20 Testpersonen vorgenommen. Die gemessenen Substrate Klebstoffe und Substrate gehen aus nachstehender Tabelle hervor:

| Bezeichnung | Klebstoff | Substrat | Beschichtung |
|---|---|---|---|
| M11.159 | Hotmelt PSA, basierend auf thermoplastischem Kautschuk, mit hoher Schäl festigkeit | PET 23my Folie | 25 gsm |
| M11.180 | Hotmelt PSA, basierend auf thermoplastischem Kautschuk mit niedriger Schälfestigkeit | PET 23my Folie | 25 gsm |
| M11.1506 | Hotmelt PSA, auf Acrylatbasis mit schwacher bis mittlerer Schälfestigkeit | PET 23my Folie | 100 gsm |
| M13.1048 | Hotmelt PSA, basierend auf thermoplastischem Kautschuk mit mittlerer Schälfestigkeit | PET 23my Folie | 25 gsm |
| Bsp. 1, glatter Träger | Basierend auf 1-Buten/Ethylen Co-Polymer | PET 23my Folie | 12-15 gsm |
| Bsp. 1, textiler Träger | Basierend auf 1-Buten/Ethylen Co-Polymer | Elastisches Gewebe aus Baumwolle und Elastan | 12-15 gsm |

Die Probestreifen wurden mit dem Klebstoff jeweils mittel Breitschlitzdüse beschichtet und die beschichteten Träger wurden 24 h bei 23°C und 50% rel. Luftfeuchtigkeit gelagert.

Danach wurde den 20 Testpersonen je ein Teststreifen pro Klebstoff unter manuellem Druck auf den Unterarm geklebt und die Teststreifen wurden 20 min auf der Haut belassen. Nach Ablauf der 20 min wurden die 6 Teststreifen sequenziell von der Haut der Testpersonen abgezogen und die Testpersonen beurteilten die Haftung qualitativ gemäss der folgenden Skala: 5 - Stärkste Haftung; 4 - Deutliche Haftung; 3 - mittlere Haftung; 2 - leichte Haftung; 1 - minimale Haftung; 0 - keine Haftung.

Die Resultate der Tests sind in Figur 1 dargestellt. Aus den Tests geht hervor, dass der Hotmelt-Klebstoff gemäss Beispiel 1 noch eine geringe Resthaftung zeigt, wenn er auf einen glatten Träger beschichtet wird. Bei keiner der 20 Testpersonen hat jedoch der auf Bandagenmaterial beschichtete Hotmelt-Klebstoff eine Haftung gezeigt.

## Patentansprüche

1. Bandage umfassend ein Flachbahnmaterial als Substrat, wobei beide Hauptseiten des Substrats zumindest teilweise mit einem Hotmelt-Klebstoff beschichtet sind, insbesondere mit einem Hotmelt-Klebstoff aufweisend ein erstes Polyolefin aus:
- einem Anteil von 1-Buten abgeleiteten Monomer-Einheiten; und
- einem Anteil von Ethylen abgeleiteten Monomer-Einheiten;
und wobei der Hotmelt-Klebstoff weiter umfasst:
- wenigstens einen Klebrigmacher aus Kohlenwasserstoffharz und/oder aus hydriertem Kohlenwasserstoffharz.

2. Bandage nach Anspruch 1, wobei der Hotmelt-Klebstoff wenigstens ein zweites Polyolefin aufweist, wobei das zweite Polyolefin bevorzugt amorph ist, besonders bevorzugt Propylen-basiert ist, besonders bevorzugt eine Mischung aus Propylen-Homopolymer und Propylen-basiertem Copolymer ist.

3. Bandage nach Anspruch 2, wobei die kombinierten Anteile des ersten und zweiten Polyolefins am Hotmelt-Klebstoff insgesamt 17 bis 43 Gew.-%, bevorzugt 20 bis 42 Gew.-%, besonders bevorzugt 25 bis 40 Gew.-% betragen.

4. Bandage nach einem der vorhergehenden Ansprüche, wobei im Hotmelt-Klebstoff das anteilige Verhältnis (in Gew.-%) von Klebrigmacher zum Gesamtanteil von Polyolefin im Bereich von 0.6 bis 3, bevorzugt im Bereich von 0.8 bis 1.8, noch mehr bevorzugt im Bereich von 1 bis 1.4 liegt.

5. Bandage nach einem der vorhergehenden Ansprüche, wobei der Hotmelt-Klebstoff weiter einen Schmiermittelzusatz umfasst, bevorzugt ein Wachs, besonders bevorzugt ein Polypropylenwachs.

6. Bandage nach Anspruch 5, wobei der relative Anteil des Schmiermittelzusatzes wenigstens 5 Gew.-%, bevorzugt 10-40 Gew.-% beträgt, noch mehr bevorzugt 20-35 Gew.-% beträgt.

7. Bandage nach einem der vorhergehenden Ansprüche, wobei der Anteil von 1-Buten abgeleiteten Monomer-Einheiten im ersten Polyolefin 80 bis 92 Gew.-% beträgt, bevorzugt 84 bis 90 Gew.-% beträgt.

8. Bandage nach einem der vorhergehenden Ansprüche, wobei der Hotmelt-Klebstoff eine Schmelzviskosität von 1'000 bis 50'000 mPa·s hat, bevorzugt 10'000 bis 45'000 mPa·s, besonders bevorzugt 15'000 bis 30'000 mPa·s, messbar nach EN ISO 3219.

9. Bandage nach einem der vorhergehenden Ansprüche, wobei der Hotmelt-Klebstoff eine Filmfestigkeit von 0.5 bis 1.5 N/mm² aufweist, bevorzugt von 0.6 bis 1.1 N/mm2, gemäss DIN 53504.

10. Bandage nach einem der vorhergehenden Ansprüche, wobei der Hotmelt-Klebstoff eine Bruchdehnung von 700 bis 1200%, bevorzugt eine Bruchdehnung von 800 bis 1000%, aufweist, messbar gemäss DIN 53504.

11. Bandage nach einem der vorhergehenden Ansprüche, wobei das Substrat ein gewebtes oder nicht-gewebtes Textil ist, bevorzugt ein in Längs- und/oder Querrichtung elastisches gewebtes oder nicht-gewebtes Textil ist.

12. Hotmelt-Klebstoff zur Herstellung einer Bandage nach einem der vorhergehenden Ansprüche, wobei der Hotmelt-Klebstoff auf einem ersten Polyolefin basiert, aufweisend
- einen Anteil von 1-Buten abgeleiteten Monomer-Einheiten;
- einem Anteil von Ethylen abgeleiteten Monomer-Einheiten;
und wobei der Hotmelt-Klebstoff weiter umfasst:
- wenigstens einen Klebrigmacher aus Kohlenwasserstoffharz und/oder aus hydriertem Kohlenwasserstoffharz;
wobei die Schälfestigkeit eines Verbunds aus zwei mit dem Hotmelt-Klebstoff beschichteten Substraten bei Raumtemperatur wenigstens 1 bis 10 N/25mm, vorzugsweise 1.5 bis 6.5 N/25mm, besonders bevorzugt 4 bis 6 N/25mm beträgt, messbar nach ASTM D 1876-01.

13. Verwendung eines Hotmelt-Klebstoffs basierend auf einem ersten Polyolefin, insbesondere aufweisend:
- einen Anteil von 1-Buten abgeleiteten Monomer-Einheiten;
- einen Anteil von Ethylen abgeleiteten Monomer-Einheiten;
wobei der Hotmelt-Klebstoff weiter umfasst:
- wenigstens einen Klebrigmacher aus Kohlenwasserstoffharz und/oder hydriertem Kohlenwasserstoffharz;
zur Herstellung von Bandagen.

14. Verfahren zur Herstellung einer Bandage umfassend die folgenden Schritte:
- Bereitstellen eines Flachbahnmaterials als Substrat, insbesondere eines in Längs- und/oder Querrichtung elastischen gewebten oder nicht-gewebten Textils;
- Bereitstellen eines Hotmelt-Klebstoffs nach Anspruch 12;
- Doppelseitiges Auftragen des Hotmelt-Klebstoffs auf das Substrat.

15. Verfahren nach Anspruch 14, wobei der Hotmelt-Klebstoff lösungsmittelfrei bereitgestellt wird.
